Europäisches Patentamt

European Patent Office

Office européen des brevets

(19)

(11)  **EP 0 891 199 B1**

(12)     **EUROPÄISCHE PATENTSCHRIFT**

(45) Veröffentlichungstag und Bekanntmachung des
Hinweises auf die Patenterteilung:
**08.09.1999 Patentblatt 1999/36**

(21) Anmeldenummer: **97917258.2**

(22) Anmeldetag: **07.03.1997**

(51) Int. Cl.$^6$: **A61M 16/00**, A61B 5/087,
A61M 16/08

(86) Internationale Anmeldenummer:
**PCT/DE97/00444**

(87) Internationale Veröffentlichungsnummer:
**WO 97/32619 (12.09.1997 Gazette 1997/39)**

(54) **VORRICHTUNG UND VERFAHREN ZUR ÜBERWACHUNG VON ATEMKENNWERTEN EINES BEATMUNGSSYSTEMS**

DEVICE AND PROCESS FOR MONITORING THE RESPIRATION PARAMETERS OF AN ARTIFICIAL RESPIRATION SYSTEM

DISPOSITIF ET PROCEDE POUR SURVEILLER DES PARAMETRES DE RESPIRATION D'UN SYSTEME DE RESPIRATION ARTIFICIELLE

(84) Benannte Vertragsstaaten:
**BE CH DE DK ES FI FR GB IT LI LU NL SE**

(30) Priorität: **08.03.1996 CH 60796**

(43) Veröffentlichungstag der Anmeldung:
**20.01.1999 Patentblatt 1999/03**

(73) Patentinhaber: **Medisize BV**
**2181 AB Hillegom (NL)**

(72) Erfinder:
• **LAMMERS, Léon**
**NL-2133 DN Hoofddorp (NL)**
• **CORNELIUS-LORENZ, Karl, Siegfried**
**D-37083 Göttingen (DE)**
• **ZÜCHNER, Klaus**
**D-37073 Göttingen (DE)**

(74) Vertreter:
**Patentanwälte Thömen & Körner**
**Zeppelinstrasse 5**
**30175 Hannover (DE)**

(56) Entgegenhaltungen:
**EP-A- 0 627 196           FR-A- 2 304 359**
**FR-A- 2 505 658           US-A- 4 581 012**

• **"Die elastische Blende als Atemstromrezeptor.**
**Grundlagen eines neuen Messprinzips."**
**Universität Fridericiana Karlsruhe Technische**
**Hochschule Manfred Franetzki aus Halle/S. Tag**
**des Kolloquiums: 2. Juli 1975 XP002034721**
• **WILLIAM W. MUSCHIN ET AL.: 'Automatic**
**Ventilation of the Lungs', 1980, BLACKWELL**
**SCIENTIFIC PUBLICATIONS, OXFORD LONDON**
**EDINBURGH MELBOURNE XP002034722 siehe**
**Seite 178, Zeile 4 - Seite 181, Zeile 2;**
**Abbildungen 7.1, 7.2**

EP 0 891 199 B1

**Beschreibung**

Die Erfindung betrifft eine Vorrichtung und ein Verfahren zur Überwachung von Atemkennwerten eines Beatmungssystems nach dem Oberbegriff des Anspruchs 1 bzw. 12.

Bei Beatmungssystemen zur künstlichen Beatmung von Patienten ist die Einhaltung von Warme- und Feuchtigkeitswerten, die der natürlichen Umgebung weitgehend entsprechen, von großer Bedeutung. Zumeist erfolgt die Versorgung mit Atemgasen aus Flaschen oder einem zentralen Versorgungssystem, bei dem die Atemgase weitgehend trocken sind. Dies würde ohne Gegenmaßnahmen zu einer Austrocknung der Atemwege des beatmeten Patienten führen.

Um die physiologisch zweckmäßigen Wärme- und Feuchtigkeitswerte einzuhalten, sind Wärme- und Feuchtigkeitstauscher, als HME bezeichnet, bekannt, die als Behandlungseinrichtung im Beatmungsschlauchsystem angeordnet sind. Diese absorbieren Wärme- und Feuchtigkeit aus den Ausatmungsgasen und setzen sie den Einatmungsgasen wieder zu. Die Behandlungseinrichtung kann auch als Filter dienen oder selbst allein als Filter ausgebildet sein, das einerseits eventuelle Verunreinigungen aus dem Beatmungssystem zurückhält und andererseits verhindert, daß Patientenkeime beim Ausatmen das Beatmungssystem verunreinigen. Steigt der Strömungswiderstand durch zu starke Verunreinigung übermäßig an, ist der Patient gezwungen, seine Atemtätigkeit zu verändern. Außerdem besteht die Gefahr, daß eine Unterversorgung mit Atemgasen eintritt. Dies stellt eine unerwünschte Belastung für den Patienten dar. Das Pflege- und Behandlungspersonal muß deshalb regelmäßig den Atemwiderstand einer solchen Behandlungseinrichtung überprüfen, um zwischenfalle zu vermeiden.

Aus der gattungsbildenden FR 23 04 359 A ist bereits eine Vorrichtung zur Überwachung von Atemkennwerten eines Beatmungssystems mit einem Wärme- und Feuchtigkeitstauscher bekannt. Hierbei wird der Strömungswiderstand des Warme- und Feuchtigkeitstauschers ausgenutzt, um über Drucksensoren vor und hinter dem Strömungswiderstand den Volumenstrom zu ermitteln. In der Druckschrift ist auch das Problem behandelt, daß sich der Strömungswiderstand des Wärme- und Feuchtigkeitstauschers in Abhängigkeit der aufgenommen Feuchtigkeit ändert. Durch diese Änderung wird die Berechnung des Volumenstroms über der Druckdifferenz des Strömungswiderstandes verfälscht. Zur Lösung dieses Problems ist vorgesehen, eine Änderung des Strömungswiderstandes zu vermeiden. Dazu wird ein Abfluß unter dem Wärme- und Feuchtigkeitstauscher angebracht, über den die kondensierte Feuchtigkeit ablaufen kann. Zwar wird im speziellen Fall kondensierter Feuchtigkeit der Atemluft eine Veränderung des Strömungswiderstandes verringert; die beschriebene Maßnahme ist aber nicht in der Lage, Veränderungen des Strömungswiderstandes zu vermeiden, wenn der Wärme- und Feuchtigkeitstauscher durch zähflüssigen Auswurf aus der Lunge verstopft wird.

Der Erfindung liegt die Aufgabe zugrunde, bei einer Vorrichtung und einem Verfahren zur Überwachung von Atemkennwerten diese automatisch mit hoher Genauigkeit und Zuverlässigkeit zu erfassen, ohne dabei die Funktionssicherheit des Beatmungssystems selbst zu beeinträchtigen.

Diese Aufgabe wird bei einer Vorrichtung nach dem Oberbegriff des Anspruchs 1 und einem Verfahren nach dem Oberbegriff des Anspruchs 12 durch die im jeweiligen Kennzeichen angegebenen Merkmale gelöst.

Weiterbildung und vorteilhafte Ausgestaltungen der Erfindung ergeben sich aus den Unteransprüchen.

Aufgrund des Zusammenhangs von Strö- mungswiderstand R gleich Druckdifferenz $\Delta p$ durch Volumenstrom $\dot{V}$ und der Beziehung Volumenstrom $\dot{V}$ gleich Volumen V durch Zeit t läßt sich eine der Größen rechnerisch ermitteln, wenn die anderen bekannt sind. Um den Strömungswiderstand der Behandlungseinrichtung zu erhalten, müssen die Druckdifferenz über der Behandlungseinrichtung und der Volumenstrom bekannt sein. Die Druckdifferenz wird dabei durch Drucksensoren vor und hinter der Behandlungseinrichtung ermittelt. Um auch den Strömungswiderstand bestimmen zu können, wird ein als Referenz geeigneter Strömungswiderstand hinzugezogen. Da dieser ebenfalls im Beatmungssystem angeordnet ist, wird er von demselben Volumenstrom durchströmt. Der Volumenstrom kann somit aus der Druckdifferenz über dem als Referenz dienenden Strömungswiderstand und dem Wert des Referenz-Strömungswiderstandes selbst bestimmt werden.

Damit läßt sich der Wert des Strömungswider- standes der Behandlungseinrichtung ständig kontrollie- ren, eine Änderung sofort ermitteln und bei Überschreitung eines Grenzwertes Alarm geben. Auch alle anderen Atemkennwerte und daraus eventuell resultierende Warnhinweise lassen sich aus den Meß- werten durch entsprechende mathematische Berech- nung ermitteln. Daher braucht das Behandlungspersonal lediglich die Überwachungseinheit zu beobachten, nicht jedoch das Beatmungssystem, insbesondere die Behandlungseinrichtung ständig zu kontrollieren.

Die Drucksensoren können Drücke innerhalb des Beatmungssystems auf eine Umgebungsreferenz bezogen ermitteln oder Druckdifferenzen über den Strömungswiderständen ermitteln. Mit Drucksensoren, die Drücke auf eine Umgebungsreferenz bezogen ermitteln, lassen sich zusätzlich die Atemwegsdrücke innerhalb des Beatmungssystems ermitteln. Mit Drucksensoren, die lediglich Druckdifferenzen ermitteln, ist ein geringerer Meßaufwand möglich.

Der als Referenz geeignete Strömungswiderstand innerhalb des Beatmungssystems kann als Abschnitt des Beatmungsschlauchsystems, als eine Blende oder als ein Filter ausgebildet sein. Bei Einbeziehung des Abschnitt des Beatmungsschlauchsystems als Referenz kommt man ohne zusätzlichen Strömungswiderstand aus. Allerdings kann der Wert dieses Strömungswiderstandes bei Verlagerung des Beatmungsschlauchsystems leicht variieren. Die weitere Möglichkeit, eine Blende zu verwenden, konzentriert zwar den Referenz-Strömungswiderstand auf einen kurzen Weg, hat aber zumeist nichtlineare Eigenschaften, die einer besonderen Kompensation bedürfen. Als optimal hat sich ein Filter als Referenz-Strömungswiderstand herausgestellt. Mit einem Filter werden nämlich der Vorteil der konzentrierten Anordnung mit dem Vorteil des linearen Verhaltens bei unterschiedlichen Strömungsgeschwindigkeiten kombiniert.

Ein als Referenz besonders geeigneter Filter ist als Vlies ausgebildet. Dies ermöglicht eine kostengünstige und mit hoher Genauigkeit reproduzierbare Herstellung.

Vorzugsweise erstreckt sich das Vlies über einen im Vergleich zum Querschnitt der Beatmungsschläuche wesentlich größeren Querschnitt. Hierdurch wird einmal die Strömungsgeschwindigkeit im Vergleich zu den Beatmungsschläuchen herabgesetzt, was Wirbelbildung und damit nichtlineare Strömungseigenschaften vermeidet und außerdem dazu beiträgt, daß bei Verunreinigung eine größere Aufnahmefläche zur Verfügung steht und daher der Strömungswiderstand weitestgehend konstant bleibt.

Bei einer vorteilhaften Ausführung ist der Filter in das Gehäuse der Behandlungseinrichtung integriert. Durch diese Maßnahme läßt sich sowohl die Anzahl der störanfälligen Steckverbindungen sowie die Länge und das Gewicht der Beatmungseinrichtung reduzieren und auch der im Beatmungssystem vorhandene Totraum einschränken, wodurch eine unerwünschte Rückatmung ausgeatmeter Gase ebenfalls vermindert wird.

Vorzugsweise münden Verbindungskanäle für die Drucksensoren in Bereiche der Behandlungseinrichtung und des als Referenz geeigneten Strömungswiderstandes innerhalb des Beatmungssystems, in denen vergleichsweise geringe Strömungsgeschwindigkeiten herrschen. Dadurch wird eine Veränderung des Druckes und damit eine Verfälschung der Meßwerte durch den Venturieffekt vermieden.

Eine Weiterbildung sieht vor, daß die Verbindungskanäle für die Drucksensoren innerhalb des Gehäuses der Behandlungseinrichtung zu einer gemeinsamen Anschlußanordnung geführt sind. Die Verbindungskanäle sind dadurch geschützt untergebracht und lassen sich von der Anschlußanordnung in kompakter Form mit den Drucksensoren verbinden.

Weiterhin kann das Beatmungsschlauchsystem zwischen der Behandlungseinrichtung und einem Beatmungsgerät in einen separaten Einatmungsschlauch und Ausatmungsschlauch unterteilt sein, dessen Zusammenführung unmittelbar vor der Behandlungseinrichtung angeordnet ist.

Besonders vorteilhaft ist ein Beatmungsschlauchsystem, bei dem der Einatmungsschlauch und der Ausatmungsschlauch zwischen der Behandlungseinrichtung und dem Beatmungsgerät koaxial ineinander liegen. Hierdurch wird der Vorteil getrennter Schläuche genutzt, ohne daß diese nach außen störend in Erscheinung treten. Außerdem ermöglicht diese Ausführung einen Wärmeaustausch.

Gemäß einer Weiterbildung bildet die Verbindung der Zusammenführung des Einatmungsschlauches und Ausatmungsschlauches mit der Behandlungseinrichtung und die Verbindung der an die Drucksensoren angeschlossenen Schläuche mit der Anschlußanordnung an der Behandlungseinrichtung eine jeweils einzeln oder gemeinsam handhabbare Steckverbindung. Die an die Drucksensoren angeschlossenen Schläuche können entlang des Beatmungsschlauchsystems geführt oder in diesen integriert sein. Hierdurch wird nicht nur der Zeitaufwand zur Montage und Demontage des Beatmungssystems wesentlich verringert, die gemeinsame Verbindung ist auch zuverlässiger und haltbarer als mehrere getrennte, örtlich unterschiedlich angeordnete Verbindungen. Darüber hinaus läßt sich hierdurch auch die Anzahl sich unkontrolliert anordnender und dadurch den Behandlungsablauf störender Schläuche und Kabel verringern.

Bedarfsweise lassen sich aus den Meßwerten auch eine Mehrzahl von Atemkenngrößen ermitteln, wie Strömungswiderstand der Behandlungseinrichtung, Tidal-Volumen, d. h. Volumen eines Atemzuges oder Atemhubes, Atem-Minuten-Volumen, Atemfrequenz, Tubusverstopfung, Airtrapping, d. h. gefangene Luft z. B. bei Asthmatikern, Atemwegsdruck oder Dichtigkeit des Beatmungssystems. Die Bereitstellung getrennter Meßgeräte und gesonderter Meßsensoren mit den entsprechenden Kabeln und Schläuchen kann daher entfallen.

Nachfolgend wird die Erfindung anhand eines Ausführungsbeispiels näher erläutert, das in der Zeichnung dargestellt ist.

In der Zeichnung zeigen:

Fig. 1    eine schematische Darstellung eines Beatmungssystems,

Fig. 2    ein Ersatzschaltbild des Beatmungssystems,

Fig. 3    einen Längsschnitt durch ein Beatmungsschlauchsystem und eine Behandlungseinrichtung mit einem integrierten Referenzfilter,

Fig. 4    einen Längsschnitt durch eine Behandlungseinrichtung mit einem integrierten Referenzfilter in einer ersten Ausführung,

Fig. 5 einen Längsschnitt durch eine Behandlungseinrichtung mit einem integrierten Referenzfilter in einer zweiten Ausführung und

Fig. 6 einen Längsschnitt durch eine Behandlungseinrichtung mit ausschließlich einem Filter.

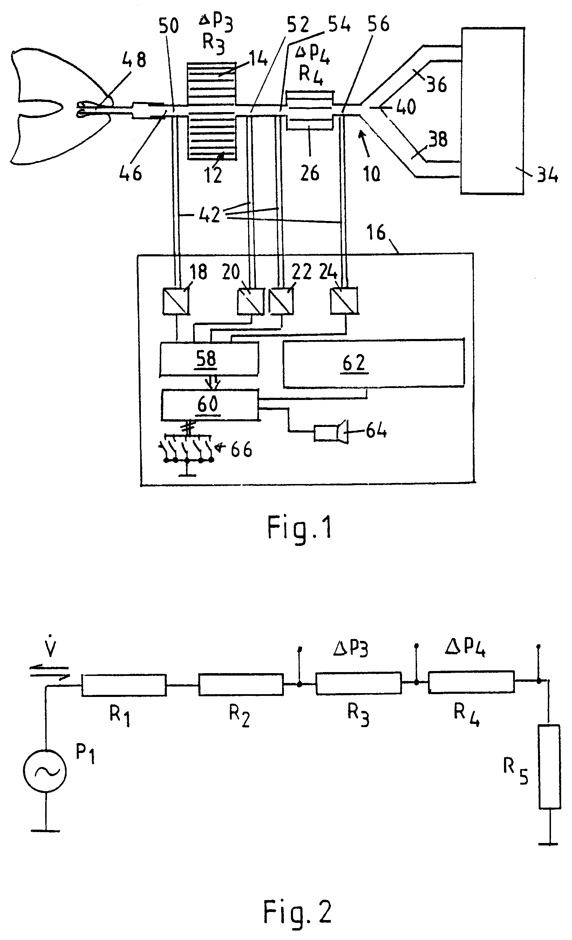

Die in Fig. 1 gezeigte schematische Darstellung eines Beatmungssystems umfaßt ein Beatmungsgerät 34, an das ein Beatmungsschlauchsystem 10 mit einer Behandlungseinrichtung 12 angeschlossen ist, einen Tubus 46, der in die Luftröhre 48 eines Patienten intubiert ist, sowie Drucksensoren 18, 20, 22, 24 und eine Überwachungseinheit 16. Die Behandlungseinrichtung 12 kann einen Wärme- und Feuchtigkeitaustauscher 14 enthalten. Der Wärme- und Feuchtigkeitsaustauscher 14 kann gleichzeitig als Filter ausgebildet sein. Alternativ ist es auch möglich, eine Behandlungseinrichtung 12 mit nur einem Filter vorzusehen. Dies hängt von einer eventuell vorhandenen unabhängigen Befeuchtungs- und Erwärmungseinrichtung ab. Zusätzlich umfaßt das Beatmungssystem noch einen weiteren als Referenz dienenden Strömungswiderstand $R_4$, der hier als zusätzlicher Filter 26 in das Beatmungsschlauchsystem 10 eingesetzt ist. Das Beatmungsschlauchsystem 10 seinerseits besteht aus einem Einatmungsschlauch 36 und einem Ausatmungsschlauch 38, die vor der Behandlungseinrichtung 12 mittels einer Zusammenführung 40 vereinigt sind.

Von Meßstellen 50, 52 vor und hinter der Behandlungseinrichtung 12 sowie Meßstellen 54, 56 vor und hinter dem Referenzfilter 26 führen dünne Meßschläuche 42 zu den Drucksensoren 18, 20, 22, 24, die hier innerhalb der Überwachungseinheit installiert sind. Die Drucksensoren 18, 20, 22, 24 ermitteln den Druck bezogen auf den atmosphärischen Umgebungsdruck. Es ist aber auch möglich, Drucksensoren vorzusehen, die nur die Differenz über der Behandlungseinrichtung 12 und über dem Referenzfilter 26 ermitteln. Ausgänge der Drucksensoren 18, 20, 22, 24 sind, gegebenenfalls über Analog-Digital-Wandler 58, mit einer Recheneinheit 60 der Überwachungseinheit 16 verbunden. An die Recheneinheit 60 sind eine Anzeigeeinheit 62, ein Alarmgeber 64 sowie Bedienungtasten 66 oder eine Tastatur angeschlossen. Zusätzlich können auch ein elektronischer Speicher und/oder ein Drucker angeschlossen sein.

In Fig. 2 ist ein Ersatzschaltbild von dem in Fig. 1 darstellten Beatmungssystem angegeben. Die Atemtätigkeit der Lungen eines Patienten ist mit einer Druckquelle $P_1$ dargestellt, die in Serie mit dem Widerstand $R_1$ der Atemwege 48, mit dem Widerstand $R_2$ des Tubus 46, mit dem Widerstand $R_3$ der Behandlungseinrichtung 12, mit dem Widerstand $R_4$ des Referenzfilters 26 und mit dem Widerstand $R_5$ des Beatmungsschlauchsystems 10 und Beatmungsgeräts 34 geschaltet ist. Durch die Atemtätigkeit wird ein mit Pfeilen dargestellter Volumenstrom $\dot{V}$ erzeugt. Über den

Widerstand $R_3$ wird eine Druckdifferenz $\Delta p_3$ und über den Widerstand $R_4$ eine weitere Druckdifferenz $\Delta p_4$ des Referenzfilters 26 erfaßt. Der Volumenstrom $\dot{V}$ beträgt dann $\dot{V} = R_4 / \Delta p_4$. Der Strömungswiderstand $R_3$ der Behandlungseinrichtung 12 ist $R_3 = \Delta p_3 / \dot{V}$. Das Volumen $V$ der vom Patienten eingeatmeten Luft errechnet sich zu $V = \int \dot{V}_{insp}\, dt$ aus dem inspiratorischen Volumenstrom $\dot{V}_{insp}$. Analoges gilt für das Volumen der ausgeatmeten Luft. Alle relevanten Atemkennwerte und deren Änderungen können so leicht ermittelt werden. Durch Vergleich mit Grenzwerten ist es außerdem möglich, kritische Zustände für den Patienten sofort zu ermitteln und zu signalisieren.

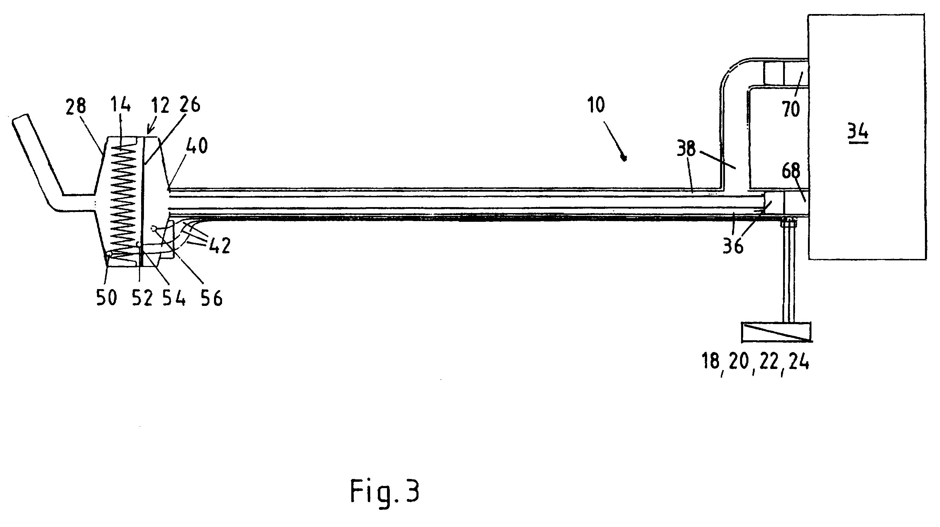

Fig. 3 zeigt einen Längsschnitt durch ein Beatmungsschlauchsystem 10 und eine Behandlungseinrichtung 12. Das Beatmungsschlauchsystem 10 besteht aus einem Einatmungsschlauch 36 und einem Ausatmungsschlauch 38 sowie einer Zusammenführung 40. Der Einatmungsschlauch 36 ist koaxial innerhalb des Ausatmungsschlauches 38 angeordnet. Anschlüsse 68, 70 des Einatmungsschlauches 36 und des Ausatmungsschlauches 38 sind mit einem Beatmungsgerät 34 verbunden. An die Zusammenführung 40 des Einatmungsschlauches 36 und Ausatmungsschlauches 38 schließt sich die Behandlungseinrichtung 12 an. Parallel zum Beatmungsschlauchsystem 10 oder in dieses integriert können sich außerdem Meßschläuche 42, die von Meßstellen 50, 52, 54, 56 zu Drucksensoren 18, 20, 22, 24 führen, befinden. Die Behandlungseinrichtung 12 umfaßt einen Wärme- und Feuchtigkeitstauscher 14 sowie einen Referenzfilter 26, der im selben Gehäuse 28 unmittelbar dem Wärme- und Feuchtigkeitstauscher 14 benachbart ist. Zur Messung der Drücke vor und hinter dem Wärme- und Feuchtigkeitstauscher 14 sowie dem Referenzfilter 26 reicht bei dieser Ausführung eine gemeinsame Meßstelle 52 zwischen dem Wärme- und Feuchtigkeitstauscher 14 einerseits und dem Referenzfilter 26 andererseits aus. Ebenso können die Drucksensoren 20 und 22 durch einen einzigen ersetzt werden.

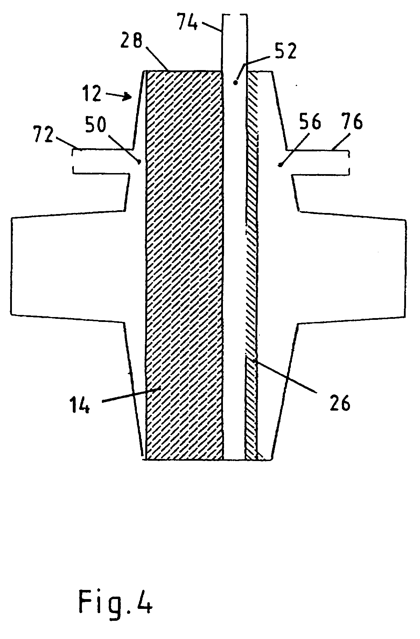

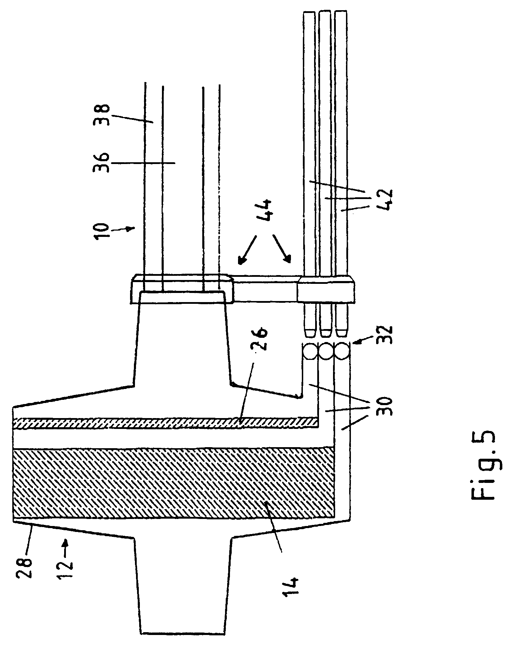

Die Figuren 4 und 5 zeigen unterschiedliche Ausgestaltungen des Wärme- und Feuchtigkeitstauscher 14 mit dem integrierten Referenzfilter 26. In Fig. 4 sind die Meßstellen 50, 52, 56 am äußeren Rand des im Querschnitt gegenüber dem Beatmungsschlauchsystem 10 vergrößerten Gehäuse 28 angeordnet. An diesen Stellen ist die Strömungsgeschwindigkeiten der Atemgase im Vergleich zur Strömungsgeschwindigkeit innerhalb des Beatmungsschlauchsystems 10 gering. Die Meßwerte werden daher nicht durch den Venturieffekt beeinflußt. Bei der Ausgestaltung nach Fig. 4 sind die Anschlüsse 72, 74, 76 unmittelbar nahe der Meßstellen 50, 52, 56 am Gehäuse 28 angebracht.

Demgegenüber zeigt Fig. 5 eine Ausgestaltung, bei der Verbindungskanäle 30 innerhalb des Gehäuses 28 der Behandlungseinrichtung 12 zu einer gemeinsamen Anschlußanordnung 32 geführt sind. Diese Anschlußanordnung 32 befindet sich auf derselben Seite des Gehäuses 28, an der auch das Beatmungsschlauchsystem 10 angeschlossen ist. Die Anschlußanordnung 32 für die Meßschläuche 42 sowie der Anschluß des Beatmungsschlauchsystems 10 ist hier in einer gemeinsamen gehandhabten Steckverbindung 44 ausgebildet. Dadurch ergeben sich keine über den Außenrand des Gehäuses 28 der Behandlungseinrichtung 12 überstehende Teile, so daß die Anschlußanordnung 32 geschützt ist. Außerdem liegen die Meßschläuche 42 bereits parallel zum Beatmungsschlauchsystem 10 und können daher ohne Biegungen oder Krümmungen in dieses integriert werden.

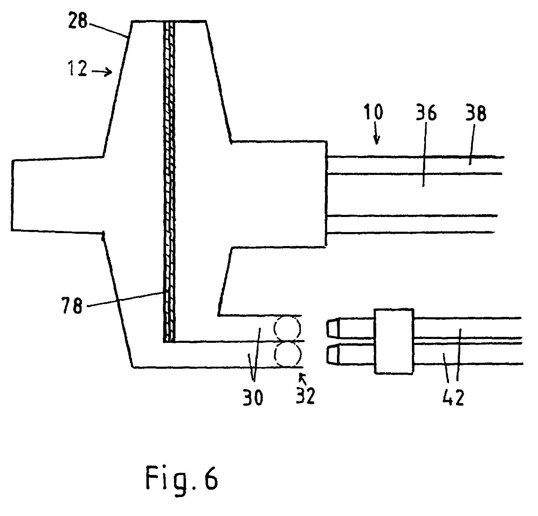

Alternativ zeigt Fig. 6 eine Ausgestaltung, bei der die Behandlungseinrichtung 12 lediglich ein Filter 78 umfaßt, daß ähnlich oder identisch dem Referenzfilter 26 ausgebildet sein kann. Wenn das Beatmungsgerät 34 für eine Wärme- und Feuchtigkeitsanpassung sorgt, kann der bei den anderen Ausführungen vorhandene Wärme- und Feuchtigkeitstauscher entfallen. Ansonsten entspricht die Ausgestaltung der Fig. 5. Mit Ausnahme einer Verstopfung des Filters 78 selbst und der davon abgeleiteten Größen lassen sich alle übrigen Atemkenngrößen mit hoher Genauigkeit ermitteln. Diese Ausführung kann somit bekannte Lösungen mit Blenden, Widerstandsleitungen oder Venturirohren ersetzen und besitzt darüber hinaus im Bereich der bei der Beatmung und Spontanatmung auftretenden Strömungsgeschwindigkeiten eine hohe Linearität.

Nachfolgend werden noch zwei Situationen erläutert, bei denen die Ermittlung der Atemkennwerte Alarmzustände kennzeichnet. 1. Das Tidal- oder Atemzugvolumen kann durch Messung der Druckdifferenz $\Delta p_4$ über dem Referenzfilter 26 und Integration über der Zeit t ermittelt werden. Ist das Integral zu gering, so ist dies ein Hinweis auf ein zu geringes Atemzugvolumen, das einen Alarmzustand darstellt. Dies kann durch ein optisches oder akustisches Signal angezeigt werden, das Anlaß zu einer sofortigen Hilfsmaßnahme gibt. 2. Der Wärme- und Feuchtigkeitstauscher 14 wird durch Schleim allmählich verstopft, wodurch dessen Widerstand $R_3$ ansteigt und somit auch die über ihm gemessene Druckdifferenz $\Delta p_3$. Um diesen Druckanstieg von einer Druckerhöhung durch einen höheren Volumenstrom unterscheiden zu können, wird zusätzlich die Druckdifferenz $\Delta p_4$ des Referenzfilters 26 ausgewertet. Durch das Verhältnis der Druckdifferenzen läßt sich dann eindeutig unterscheiden, ob der Druckanstieg durch eine Verstopfung des Wärme- und Feuchtigkeitstauschers 14 oder durch eine Erhöhung des Volumenstroms erzeugt wird und so bedarfsweise Alarm ausgelöst werden.

**Patentansprüche**

1. Vorrichtung zur Überwachung von Atemkennwerten eines Beatmungssystems mit einer in einem Beatmungsschlauchsystem (10) angeordneten Behandlungseinrichtung (12) aus einem Filter und/oder einem Wärme- und Feuchtigkeitstauscher (14) durch Auswertung von Druck- und/oder Strömungsgrößen des Beatmungssystems mittels einer Überwachungseinheit (16), wobei Drucksensoren (18, 20) vor und hinter einem Strömungswiderstand ($R_3$) der Behandlungseinrichtung (12) angeordnet sind, dadurch gekennzeichnet, daß in Serie zum Strömungswiderstand ($R_3$) der Behandlungseinrichtung (12) ein weiterer, als Referenz geeigneter Strömungswiderstand ($R_4$) innerhalb des Beatmungsschlauchsystems angeordnet ist und Drucksensoren (22, 24) ebenfalls vor und hinter diesem weiteren als Referenz geeigneten Strömungswiderstand ($R_4$) angeordnet sind, daß die Signale der Drucksensoren (18, 20, 22, 24) der Überwachungseinheit (16) zugeführt sind und daß die Überwachungseinheit (16) die Atemkennwerte aus der Druckdifferenz $\Delta p_3$ über dem Strömungswiderstand ($R_3$) der Behandlungseinrichtung (12) und der Druckdifferenz $\Delta p_4$ über dem weiteren als Referenz geeigneten Strömungswiderstand ($R_4$) innerhalb des Beatmungssystems sowie der Zeit t ermittelt.

2. Vorrichtung nach Anspruch 1, dadurch gekennzeichnet, daß die Drucksensoren (18, 20, 22, 24) Drücke innerhalb des Beatmungssystems auf eine Umgebungsreferenz bezogen ermitteln oder Druckdifferenzen über den Strömungswiderständen ($R_2$ $R_4$) ermitteln.

3. Vorrichtung nach Anspruch 1 oder 2, dadurch gekennzeichnet, daß ein als Referenz geeigneter Strömungswiderstand ($R_4$) innerhalb des Beatmungssystems als ein Abschnitt des Beatmungsschlauchsystems, als eine Blende oder als ein Filter (26) ausgebildet ist.

4. Vorrichtung nach Anspruch 3, dadurch gekennzeichnet, daß der als Referenz dienende Filter (26) als Vlies ausgebildet ist.

5. Vorrichtung nach Anspruch 4, dadurch gekennzeichnet, daß das Vlies sich über einen im Vergleich zum Querschnitt des Beatmungsschlauchsystems (10) wesentlich größeren Querschnitt erstreckt.

6. Vorrichtung nach einem der Ansprüche 3 bis 5, dadurch gekennzeichnet, daß der Filter (26) in das Gehäuse (28) der Behandlungseinrichtung (12) integriert ist.

7. Vorrichtung nach einem der Ansprüche 1 bis 6, dadurch gekennzeichnet, daß Verbindungskanäle (30) für die Drucksensoren (18, 20, 22, 24) in Bereiche der Behandlungseinrichtung (12) und des als Referenz geeigneten Strömungswiderstandes ($R_4$) innerhalb des Beatmungssystems münden, in denen vergleichsweise geringe Strömungsgeschwindigkeiten herrschen.

8. Vorrichtung nach Anspruch 7, dadurch gekennzeichnet, daß die Verbindungskanäle (30) für die Drucksensoren (18, 20, 22, 24) innerhalb des Gehäuses (28) der Behandlungseinrichtung (12) zu einer gemeinsamen Anschlußanordnung (32) geführt sind.

9. Vorrichtung nach einem der Ansprüche 1 bis 8, dadurch gekennzeichnet, daß das Beatmungsschlauchsystem (10) zwischen der Behandlungseinrichtung (12) und einem Beatmungsgerät (34) in einen separaten Einatmungsschlauch (36) und Ausatmungsschlauch (38) unterteilt ist, dessen Zusammenführung (40) unmittelbar vor der Behandlungseinrichtung (12) angeordnet ist.

10. Vorrichtung nach Anspruch 9, dadurch gekennzeichnet, daß das Beatmungsschlauchsystem (10) zwischen der Behandlungseinrichtung (12) und dem Beatmungsgerat (24) mit dem separaten Einatmungsschlauch (36) und Ausatmungsschlauch (38) koaxial ausgebildet ist.

11. Vorrichtung nach einem der Ansprüche 1 bis 10, dadurch gekennzeichnet, daß die Verbindung der Zusammenführung (40) des Einatmungsschlauches (36) und Ausatmungsschlauches (38) mit der Behandlungseinrichtung (12) und die Verbindung der an die Drucksensoren (18, 20, 22, 24) angeschlossenen Meßschläuche (42) mit der Anschlußanordnung (32) an der Behandlungseinrichtung (12) gesondert handhabbare Steckverbindungen oder eine gemeinsam handhabbare Steckverbindung (44) bilden und die an die Drucksensoren (18, 20, 22, 24) angeschlossenen Meßschläuche (42) entlang des Beatmungsschlauchsystems (10) geführt oder in diesen integriert sind.

12. Verfahren zur Überwachung von Atemkennwerten eines Beatmungssystems mit einer in einem Beatmungsschlauchsystem angeordneten Behandlungseinrichtung aus einem Filter und/oder einem Wärme- und Feuchtigkeitstauscher durch Auswertung von Druck- und/oder Strömungsgrößen des Beatmungssystems mittels einer Überwachungseinheit, wobei Drücke vor und hinter einem Strömungswiderstand der Behandlungseinrichtung oder eine Druckdifferenz über dem Strömungswiderstand der Behandlungseinrichtung ausgewertet werden, dadurch gekennzeichnet, daß zusätzlich Drücke vor und hinter einem weiteren, als Referenz geeigneten Strömungswiderstand innerhalb des Beatmungsschlauchsystems oder eine Druckdifferenz über dem als Referenz geeigneten Strömungswiderstand innerhalb des Beatmungsschlauchsystems gemessen werden, der in Serie zum Strömungswiderstand der Behandlungseinrichtung liegt, und daß außerdem Zeiten gemessen werden und aus diesen Meßgrößen die Atemkennwerte ermittelt, angezeigt und/oder gespeichert und/oder mit Grenzwerten verglichen und zur Auslösung von Alarm ausgewertet werden.

13. Verfahren nach Anspruch 12, dadurch gekennzeichnet, daß zur Gewinnung des Referenz-Strömungswiderstandes die Drücke vor und hinter oder die Druckdifferenz über einem Abschnitt des Beatmungsschlauchsystems, einer Blende oder einem Filter gemessen werden.

14. Verfahren nach Anspruch 12 oder 13, dadurch gekennzeichnet, daß die Drücke oder Druckdifferenzen in Bereichen des Beatmungssystems gemessen werden, in denen geringe Strömungsgeschwindigkeiten herrschen.

15. Verfahren nach einem der Ansprüche 12 bis 14, dadurch gekennzeichnet, daß als Atemkennwerte aus den Drücken und/oder Druckdifferenzen sowie der Zeit einer oder mehrere der folgenden Kennwerte ermittelt werden: Strömungswiderstand der Behandlungseinrichtung, Tidal-Volumen, d. h. Volumen eines Atemzuges oder Atemhubes, AtemMinuten-Volumen, Atemfrequenz, Tubusverstopfung, Airtrapping, d. h. gefangene Luft z. B. bei Asthmatikern, Atemwegsdruck oder Dichtigkeit des Beatmungssystems.

**Claims**

1. Device for monitoring respiratory characteristics of a respiratory system with a treatment facility (12), consisting of a filter and/or a heat and moisture exchanger (14) and positioned in a respiratory tube system (10), through evaluation of pressure and/or flow values of the respiratory system by means of a monitoring unit (16), whereby pressure sensors (18, 20) are positioned before and after a flow resistance ($R_3$) of the treatment facility (12), characterised in that a further flow resistance ($R_4$), suitable as reference, is placed in series to the flow resistance ($R_3$) of the treatment facility (12) within the respiratory tube system and pressure sensors (22, 24) are also positioned before and after this further flow resistance ($R_4$) which is suitable as reference, that the signals of the pressure sensors (18, 20, 22, 24) are fed to the monitoring unit (16) and that the monitoring unit (16) determines the respiratory characteristics from the pressure difference $\Delta p_3$ via the flow resistance ($R_3$) of the treatment facility (12) and the pressure difference $\Delta p_4$ via the further flow resistance ($R_4$) which is suitable as reference within the respiratory system, as well as determining the time t.

2. Device according to Claim 1, characterised in that the pressure sensors (18, 20, 22, 24) determine pressures within the respiratory system with reference to an ambient reference or determine pressure differences via the flow resistances ($R_3$ $R_4$).

3. Device according to Claim 1 or 2, characterised in that a flow resistance ($R_4$) which is suitable as reference is modelled within the respiratory system as a section of the respiratory tube system, as a screen or as a filter (26).

4. Device according to Claim 3, characterised in that the filter (26) acting as reference is modelled as a fleece.

5. Device according to Claim 4, characterised in that the fleece extends across a cross-section which is significantly larger than the cross-section of the respiratory tube system (10).

6. Device according to one of Claims 3 to 5, characterised in that the filter (26) is integrated into the casing (28) of the treatment facility (12).

7. Device according to one of Claims 1 to 6, characterised in that duct connectors (30) for the pressure sensors (18, 20, 22, 24) lead into areas of the treatment facility (12) and of the flow resistance ($R_4$) which is suitable as a reference within the respiratory system in which comparatively low flow rates dominate.

8. Device according to Claim 7, characterised in that the duct connectors (30) for the pressure sensors (18, 20, 22, 24) are fed within the casing (28) of the treatment facility (12) to a common connection arrangement (32).

9. Device according to one of Claims 1 to 8, characterised in that the respiratory tube system (10) is divided between the treatment facility (12) and a respiratory device (34) into a separate in-breath tube (36) and out-breath tube (38) whose junction (40) is situated directly in front of the treatment facility (12).

10. Device according to Claim 9, characterised in that the respiratory tube system (10) is coaxially formed between the treatment facility (12) and the respiratory device (24) with the separate in-breath tube (36) and out-breath tube (38).

11. Device according to one of Claims 1 to 10, characterised in that the connection of the junction (40) of the in-breath tube (36) and the out-breath tube (38) to the treatment facility (12) and the connection of the measuring tubes (42), which are connected to the pressure sensors (18, 20, 22, 24), to the connection arrangement (32) on the treatment facility (12) take the form of separately manipulable plug-in connections (44) or a commonly manipulable plug-in connection (44) and the measuring tubes (42) connected to the pressure sensors (18, 20, 22, 24) are led along the respiratory tube system (10) or integrated in this.

12. Method for monitoring respiratory characteristics of a respiratory system with a treatment facility, consisting of a filter and/or a heat and moisture exchanger and positioned in a respiratory tube system, through evaluation of pressure and/or flow values of the respiratory system by means of a monitoring unit, whereby pressures are evaluated before and after a flow resistance of the treatment facility, or a pressure difference is evaluated via the flow resistance of the treatment facility characterised in that, in addition, pressures are measured before and after a further flow resistance, suitable as reference, within the respiratory tube system or a pressure difference is measured via the flow resistance, suitable as reference, within the respiratory tube system which is placed in series to the flow resistance of the treatment facility and that, moreover, times are measured and from these measured values the respiratory characteristics are determined, displayed and/or stored and/or compared with limit values and evaluated to trigger an alarm.

13. Method according to Claim 12, characterised in that, to obtain the reference flow resistance, the pressures before and after or the pressure difference across a section of the respiratory tube system, a screen or a filter are measured.

14. Method according to Claim 12 or 13, characterised in that the pressures or pressure differences are measured in areas of the respiratory system in which low flow rates dominate.

15. Method according to one of Claims 12 to 14, characterised in that, as respiratory characteristics, one or more of the following characteristics is determined from the pressures and/or pressure differences and the time: flow resistance of the treatment facility, tidal volumes, i.e. volumes of an in-breath or out-breath, breath/minute volumes, respiratory frequency, tube blockage, air trapping, i.e. trapped air, e.g. in the case of asthmatics, respiratory tract pressure or non-leakage of the respiratory system.

## Revendications

1. Dispositif de surveillance des paramètres de respiration d'un système de respiration artificielle comportant un appareil de traitement (12) situé dans un système de tubes de respiration artificielle (10) et constitué d'un filtre et/ou d'un échangeur de chaleur et d'humidité (14), par évaluation de paramètres de pression et/ou de débit du système de respiration artificielle au moyen d'une unité de surveillance (16), des capteurs de pression (18, 20) étant installés en amont et en aval d'une résistance hydraulique (R$_3$) de l'appareil de traitement (12), **caractérisé en ce** que sont montés, en série avec la résistance hydraulique (R$_3$) de l'appareil de traitement (12), une autre résistance hydraulique (R$_4$), considérée comme référence, à l'intérieur du système de tubes de respiration artificielle, et des capteurs de pression (22, 24) montés, de même, en amont et en aval de cette autre résistance hydraulique (R$_4$), de façon que les signaux des capteurs de pression (18, 20, 22, 24) soient envoyés à l'unité de surveillance (16) et que l'unité de surveillance (16) détermine les paramètres à partir de la différence de pression $\Delta p_3$ au-dessus de la résistance hydraulique (R$_3$) de l'appareil de traitement (12) et de la différence de pression $\Delta p_4$ au-dessus de l'autre résistance hydraulique (R$_4$), à l'intérieur du système de respiration artificielle, ainsi qu'à partir du temps t.

2. Dispositif suivant la revendication 1, **caractérisé en ce** que les capteurs de pression (18, 20, 22, 24) déterminent les pressions à l'intérieur du système de respiration artificielle par rapport à une référence de l'environnement, ou déterminent les différences de pression au-dessus des résistances hydrauliques (R$_3$, R$_4$).

3. Dispositif suivant la revendication 1 ou 2, **caractérisé en ce** qu'une résistance hydraulique (R$_4$), considérée comme référence, à l'intérieur du système de respiration artificielle est prévue, comme une partie du système de tubes de respiration artificielle, sous forme d'un écran ou d'un filtre (26).

4. Dispositif suivant la revendication 3, **caractérisé en ce** que le filtre (26), servant de référence, est prévu sous la forme d'un non-tissé.

5. Dispositif suivant la revendication 4, **caractérise en ce** que le non-tissé s'étend sur un section comparativement grande par rapport à la section du système de tubes de respiration artificielle (10).

6. Dispositif suivant la revendication 1, **caractérisé en ce** que le filtre (26) est intégré dans l'enceinte (28) de l'appareil de respiration artificielle (12).

7. Dispositif suivant une des revendications 1 à 6, **caractérisé en ce** que des canaux de liaison (30), pour les capteurs (18, 20, 22, 24), débouchent, à l'intérieur du système de respiration artificielle, dans la zone de l'appareil de respiration artificielle (12) et de la résistance hydraulique (R$_4$), dans laquelle règnent des vitesses de débit comparativement faibles.

8. Dispositif suivant la revendication 7, **caractérisé en ce** que les canaux de liaison (30), pour les capteurs de pression (18, 20, 22, 24), sont dirigés, à l'intérieur de l'enceinte (28) de l'appareil de traitement (12), vers un dispositif de raccordement collectif (32).

9. Dispositif suivant l'une des revendications 1 à 8, **caractérisé en ce** que le système de tubes de respiration artificielle (10) est, entre l'appareil de traitement (12) et un appareil de respiration artificielle (34), divisé en un tuyau d'aspiration (36) et un tuyau d'expiration (38), dont la jonction (40) se trouve immédiatement devant l'appareil de traitement (12).

10. Dispositif suivant la revendication 9, **caractérisé en ce** que le système de tubes de respiration artificielle (10) est, entre l'appareil de traitement (12) et l'appareil de respiration artificielle (24), est monté coaxial au tuyau d'aspiration (36) et au tuyau d'expiration (38).

11. Dispositif suivant l'une des revendications 1 à 10, **caractérisé en ce** que la liaison de la jonction (40) du tuyau d'aspiration (36) et du tuyau d'expiration (38) avec l'appareil de traitement (12) et la liaison des tuyaux de mesure (42) reliés aux capteurs de pression (18, 20, 22, 24) avec la jonction (32) forment des fiches de raccordement manipulables individuelles ou une fiche de raccordement manipulable commune vers l'appareil de traitement (12), et les tuyaux de mesure (42) reliés aux capteurs (18, 20, 22, 24) sont placés le long du système de tubes de respiration artificielle (10), ou intégrés à celui-ci.

12. Procédé de surveillance des paramètres de respiration d'un système de respiration artificielle comportant un appareil de traitement placé dans un système de tubes de respiration artificielle et consistant en un filtre et/ou un échangeur de chaleur et d'humidité, fonctionnant par évaluation de valeurs de pression et/ou de débit du système de respiration artificielle, au moyen d'une unité de surveillance, les pression étant mesurées en amont et en aval d'une résistance hydraulique de l'appareil de traitement ou une différence de pressions au-dessus de la résistance hydraulique de l'appareil de traitement, **caractérisé en ce** qu'on mesure des pressions additionnelles en amont et en aval d'une autre résistance hydraulique, servant de référence, à l'intérieur du système de tubes de respiration artificielle, ou une différence de pressions au-dessus de l'autre résistance hydraulique, servant de référence, à l'intérieur du système de tubes de respiration artificielle, qui est montée en série avec la résistance hydraulique de l'appareil de traitement et en ce qu'en outre on mesure des temps, et qu'à partir de ces valeurs de mesure, les paramètres sont déterminés, affichés et/ou mis en mémoire et/ou comparés avec des valeurs limites, et sont exploités pour déclencher une alarme.

13. Procédé suivant la revendication 12, **caractérisé en ce** que, pour obtenir la résistance hydraulique de référence, on mesure les pressions en amont et en aval, ou la différence de pressions au-dessus d'une partie du système de tubes de respiration artificielle, d'un écran ou d'un filtre.

14. Procédé suivant la revendication 12 ou 13, **caractérisé en ce** qu'on mesure les pressions ou la différence de pressions dans la zone du système de respiration artificielle où règnent de faibles vitesses d'écoulement.

15. Procédé suivant l'une des revendications 12 à 14, **caractérisé en ce** que, comme paramètres obtenus a partir des pressions et/ou de différence de pressions ainsi que du temps, on détermine une ou plusieurs des valeurs caractéristiques suivantes : résistance hydraulique de l'appareil de traitement, volumes cycliques d'échange, c'est-à-dire les volumes d'une expiration ou d'une aspiration, volumes de respiration par minute, fréquence de respiration, obstruction de tube, piégeage d'air, c'est-à-dire air emprisonné par exemple par des asthmatiques, compression de voie respiratoire ou imperméabilité du système de respiration artificielle.

Fig.1

Fig.2

Fig.3

EP 0 891 199 B1

Fig.4

EP 0 891 199 B1

Fig.5

Fig. 6